(19) Europäisches Patentamt
European Patent Office
Office européen des brevets

(11) Veröffentlichungsnummer: **0 059 373**
**B1**

(12) # EUROPÄISCHE PATENTSCHRIFT

(45) Veröffentlichungstag der Patentschrift: **23.05.84**

(51) Int. Cl.³: **C 07 C 43/23**, C 07 C 43/295, C 07 C 41/28

(21) Anmeldenummer: **82101205.1**

(22) Anmeldetag: **18.02.82**

(54) **Verfahren zur Herstellung von Benzylalkoholen.**

(30) Priorität: **26.02.81 DE 3107147**

(43) Veröffentlichungstag der Anmeldung: **08.09.82 Patentblatt 82/36**

(45) Bekanntmachung des Hinweises auf die Patenterteilung: **23.05.84 Patentblatt 84/21**

(84) Benannte Vertragsstaaten: **BE CH DE FR GB IT LI NL**

(56) Entgegenhaltungen:
**DE-A- 2 019 072**
**US-A- 2 165 962**

(73) Patentinhaber: **BASF Aktiengesellschaft
Carl-Bosch-Strasse 38
D-6700 Ludwigshafen (DE)**

(72) Erfinder: **Barl, Manfred, Dr.
Pappelstrasse 2
D-6701 Otterstadt (DE)**
Erfinder: **Siegel, Hardo, Dr.
Hans-Purrmann-Allee 25
D-6720 Speyer (DE)**
Erfinder: **Degner, Dieter, Dr.
Kurpfalzstrasse 8
D-6701 Dannstadt-Schauernheim (DE)**
Erfinder: **Mercker, Hans Jochen, Dr.
Schwabenstrasse 14
D-6800 Mannheim (DE)**

**EP 0 059 373 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Diese Erfindung betrifft ein neues Verfahren zur Herstellung von Benzylalkoholen, die in 4-Stellung einen Alkoxy-, Phenoxy- oder Aralkoxyrest enthalten.

4-Alkoxybenzylalkohole sind wertvolle Zwischenprodukte für die Herstellung von Pharmazeutika und Aromastoffen. Sie werden bekanntlich durch Hydrierung der entsprechenden 4-Alkoxybenzaldehyde (US-PS 3 663 626) oder durch Verseifung der entsprechenden Benzylhalogenide hergestellt (Ullmanns Enzyklopädie der technischen Chemie, 4. Aufl., Bd. 8, S. 436).

Diese Verfahren haben verschiedene Nachteile. So sind die Aldehyde als Ausgangsstoffe wenig lagerstabil und neigen zu Verfärbungen. Durch Autoxidation gehen die Aldehyde leicht in Benzoesäuren über, welche wiederum die Lebensdauer der für die Hydrierung benötigten heterogenen Katalysatoren beeinträchtigen. Die Aldehyde befinden sich außerdem bereits auf einem relativ hohen Veredlungsniveau und sind deshalb nicht mehr als kostengünstiges Einsatzmaterial zu bezeichnen.

Die Herstellung der Benzylhalogenide durch Chlorierung der Methylaromaten sowie die Verseifung zu den Benzylalkoholen sind umweltbelastende Methoden. Die Benzylhalogenide selbst sind hochtoxisch und wegen ihrer leichten Zersetzbarkeit nur in speziellen Apparaturen zu handhaben. Spuren an Halogenverbindungen sind aus den Benzylalkoholen kaum zu entfernen, sie beeinträchtigen bereits in ppm-Mengen die Qualität der Folgeprodukte hinsichtlich ihrer toxikologischen, organoleptischen und sensorischen Eigenschaften erheblich.

Es bestand daher die Aufgabe, ein neues Verfahren zur Herstellung von Benzylalkoholen zu suchen, welches die genannten Nachteile nicht aufweist.

Diese Aufgabe wird durch das erfindungsgemäße Verfahren gelöst. Gegenstand der Erfindung ist ein Verfahren zur Herstellung von Benzylalkoholen der Formel

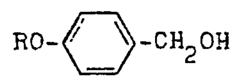

(I)

in der R einen Alkylrest mit 1 bis 8 C-Atomen, einen gegebenenfalls durch Halogenatome, Alkylgruppen oder durch Alkoxygruppen substituierten Phenylrest oder einen Aralkylrest bedeutet, bei dem man Benzaldehyddialkylacetale der Formel

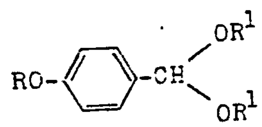

(II)

in der $R^1$ einen Alkylrest mit 1 bis 3 C-Atomen bedeutet, in Wasser und in Gegenwart eines Übergangsmetalles der 8. Nebengruppe als Katalysator bei höherer Temperatur und erhöhtem Druck hydriert.

Die als Ausgangsstoffe für das neue Verfahren zu verwendenden Benzaldehyddialkylacetale der Formel I können z. B. nach dem in der DE-OS 28 48 397 beschriebenen Verfahren dadurch hergestellt werden, daß man 4-Alkoxytoluole in Gegenwart von Alkoholen elektrochemisch oxidiert.

Als Alkylreste R mit 1 bis 8 C-Atomen kommen z. B. Methyl-, Ethyl-, Propyl-, tert.-Butyl- oder Octylgruppen in Betracht. R kann auch ein Phenylrest sein, der durch Halogenatome, wie Chloratome, durch Alkylgruppen, wie Methyl- oder Ethylgruppen oder durch Alkoxygruppen, wie Methoxy- oder Ethoxygruppen substituiert sein kann. Als ein Aralkylrest kommt z. B. der Benzylrest in Betracht. Geeignete Ausgangsstoffe der Formel II sind z. B.: 4-Methoxy-benzaldehyddimethylacetal, 4-Ethoxybenzaldehyddimethylacetal, 4-tert.-Butoxybenzaldehyddimethylacetal, 4-Octyloxybenzaldehyddi-n-propylacetal, 4-Benzyloxybenzaldehyddimethylacetal und 4-Phenoxybenzaldehyddiethylacetal.

Die 4-Alkoxybenzaldehyddialkylacetale der Formel II werden in Wasser und in Gegenwart eines der genannten Übergangsmetalle als Katalysatoren bei höherer Temperatur, z. B. bei Temperaturen von 40 bis 140 °C, vorzugsweise 60 bis 120 °C und unter erhöhtem Druck, z. B. bei 20 bis 400 bar, vorzugsweise 80 bis 250 bar hydriert. Das Wasser wird in mindestens 1-molarer Menge, bezogen auf die 4-Alkoxybenzaldehyddialkylacetale, eingesetzt. Zweckmäßigerweise verwendet man die 3 bis 20fache molare Menge Wasser, bezogen auf das Dialkylacetal. Als Katalysatoren verwendet man die als Katalysatoren für Hydrierungen üblichen Übergangsmetalle der 8. Nebengruppe. Besonders geeignet sind Eisen, Kobalt und Nickel.

Die erfindungsgemäße Hydrierung wird besonders vorteilhaft in Abwesenheit von Säuren und von organischen Lösungsmitteln durchgeführt. Daß die Hydrierung unter diesen Bedingungen erfolgreich durchgeführt werden kann, ist überraschend, da sich in Wasser relativ wenig Wasserstoff löst, und Wasser für Hydrierungen ein ungebräuchliches Lösungsmittel darstellt, wenn man von Spezialfällen absieht, bei denen durch Zusatz von Säuren besondere Aktivierungseffekte erzielt werden. Da weder die Alkoxybenzaldehydacetale, noch die Alkoxybenzaldehyde und auch nicht die Alkoxybenzylalkohole wasserlöslich sind und somit bei der Hydrierung ein 4-Phasensystem vorliegt (Gas, fester Katalysator, 2 Flüssigkeitsphasen), bei dem sich bekanntlich Schwierigkeiten bei der Durchmischung und bei

Phasenübergängen ergeben, konnte nicht erwartet werden, daß sich die Alkoxybenzaldehyddialkylacetale nach dem erfindungsgemäßen Verfahren so glatt zu den Alkoxybenzylalkoholen hydrieren lassen. Es ist zwar bekannt, daß Acetale mit Wasser leicht zu Aldehyden umgesetzt werden, jedoch wird hierfür die Gegenwart katalytischer Mengen Säure benötigt. In Houben-Weyl, Methoden der organischen Chemie, Bd. 6/3, S. 274, wird z. B. empfohlen, niedrigmolekulare Acetale durch Kochen mit 1 bis 2 %iger Salzsäure und höhermolekulare Acetale durch Behandeln mit 5 bis 10 %iger Säure zu verseifen. Eine Reihe weiterer als Katalystoren geeignete Säuren werden in Bd. 7/1, S. 423 genannt. In der EP 0 012 240, S. 21 wird beschrieben, daß 4-Methoxybenzaldehyddimethylacetal in Gegenwart eines sauren Ionenaustauschers oder der starken Säure p-Toluolsulfonsäure verseift wird. Eine solche Säure würde aber den für die Hydrierung benötigten Katalysator irreversibel schädigen, wollte man versuchen, das Benzaldehyddimethylacetal in dem System aus Acetal, Wasser und Säure zu hydrieren.

Nach dem erfindungsgemäßen Verfahren erhält man die 4-Alkoxybenzylalkohole der Formel I glatt und in guten Ausbeuten. Die 4-Alkoxybenzylalkohole sind wertvolle Zwischenprodukte für die Herstellung von Pharmazeutika oder Aromastoffen. Beispielsweise können sie für die Herstellung des Antitussivum, Dextromethorphan oder des Herbizides 4-Methoxybenzyl-N,N-diethylthiolcarbamat verwendet werden.

Beispiel 1

In einem 300 ml Autoklaven, der mit einem Begasungsrührer ausgerüstet ist, werden 100 g 4-Methoxybenzaldehyddimethylacetal, 50 g Wasser und 10 g Raney-Kobalt bei 100 °C mit 100 bar Wasserstoff behandelt. Es wird stündlich die verbrauchte Menge Wasserstoff ersetzt. Nach 4 Stunden ist die Reaktion beendet, es werden ca. 260 bar aufgenommen. Das Gaschromatogramm des Reaktionsproduktes zeigt folgende Zusammensetzung :

0,4 Gew.% 4-Methylanisol
2,3 Gew.% 4-Methoxybenzylmethylether
< 0,1 Gew.% 4-Methoxybenzaldehyddimethylacetal
1,6 Gew.% 4-Methoxybenzaldehyd
95,6 Gew.% 4-Methoxybenzylalkohol
1,4 Gew.% unbekannte Bestandteile

Beispiel 2

In einem 5 l-Autoklaven, der mit einem Hubrührer ausgerüstet ist, werden 800 g 4-Methoxibenzaldehyddimethylacetal 400 ml Wasser und 80 g Raney-Nickel auf 80 °C erhitzt. Wenn die Innentemperatur ca. 2 Stunden konstant ist, wird langsam Wasserstoff aufgepreßt, bis ein Innendruck von 100 bar erreicht ist. Der verbrauchte Wasserstoff wird stündlich ersetzt. Die Reaktion ist nach 4 Stunden und einer Gasaufnahme von 102 bar beendet.

Das Reaktionsprodukt hat folgende Zusammensetzung :

0,8 Gew.% 4-Methylanisol
1,2 Gew.% 4-Methoxybenzylmethylether
< 0,1 Gew.% 4-Methoxybenzaldehyddimethylacetal
1,4 Gew.% 4-Methoxybenzaldehyd
96,3 Gew.% 5-Methoxybenzylalkohol
0,4 Gew.% unbekannte Bestandteile

Beispiel 3

In einem 300 ml Autoklaven werden 120 g 4-Ethoxybenzaldehyddiethylacetal, 50 ml Wasser und 10 g Raney-Kobalt bei 100 °C mit 200 bar Wasserstoff behandelt. Die verbrauchte Menge Wasserstoff wird stündlich ersetzt. Nach 3 Stunden ist die Reaktion beendet. Es werden ca. 300 bar Wasserstoff aufgenommen. Das Gaschromatogramm des Reaktionsproduktes zeigt, daß 4-Ethoxybenzylalkohol in einer Ausbeute von 94 % entstanden ist.

Beispiel 4

Man verfährt wie in Beispiel 3 beschrieben, wobei man aber anstelle des 4-Ethoxybenzaldehyddimethylacetals die gleiche Menge 4-tert.-Butoxybenzaldehyddimethylacetal einsetzt. Die Reaktionsdauer beträgt 5 Stunden bei einer Gasaufnahme von 280 bar. Die gaschromatographisch bestimmte Ausbeute an 4-tert.-Butoxybenzalkohol beträgt 91 %.

Beispiel 5

In einem 300 ml Autoklaven werden 150 g 4-Benzyloxybenzaldehyddimethylacetal, 40 ml Wasser und

10 g Raney-Kobalt auf 120 °C erhitzt. Wenn die Temperatur ca. 2 Stunden konstant ist, wird langsam Wasserstoff aufgepreßt, bis ein Druck von 80 bar erreicht ist. Der verbrauchte Wasserstoff wird stündlich ersetzt, die Umsetzung ist nach 5 Stunden beendet. Das Gaschromatogramm zeigt, daß 4-Benzyloxybenzylalkohol mit einer Ausbeute von 93 % entstanden ist.

Beispiel 6

In einem 500 ml Autoklaven werden 150 g 4-Phenoxybenzaldehyddimethylacetal, 100 ml Wasser und 15 g Raney-Kobalt bei 100 °C mit 250 bar Wasserstoff behandelt. Der verbrauchte Wasserstoff wird stündlich ersetzt. Die Reaktion ist nach 7 Stunden beendet. Die gaschromatographisch ermittelte Ausbeute von 4-Phenoxybenzylalkohol beträgt 89 %.

**Ansprüche**

1. Verfahren zur Herstellung von Benzylalkoholen der Formel

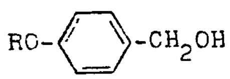

(I)

in der R einen Alkylrest mit 1 bis 8 C-Atomen, einen gegebenenfalls durch Halogenatome, Alkylgruppen oder durch Alkoxygruppen substituierten Phenylrest oder einen Aralkylrest bedeutet, dadurch gekennzeichnet, daß man Benzaldehyddialkylacetale der Formel

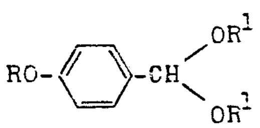

(II)

in der $R^1$ einen Alkylrest mit 1 bis 3 C-Atomen bedeutet, in Wasser und in Gegenwart eines Übergangsmetalles der 8. Nebengruppe als Katalysator bei höherer Temperatur und erhöhtem Druck hydriert.

2. Verfahren nach Anspruch 1, dadurch gekennzeichnet, daß man die Hydrierung bei Temperaturen von 40 bis 140 °C und Drücken von 20 bis 400 bar vornimmt.

3. Verfahren nach Anspruch 1, dadurch gekennzeichnet, daß man als Katalysator Eisen, Kobalt oder Nickel verwendet.

**Claims**

1. A process for the preparation of a benzyl alcohol of the formula

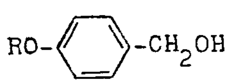

(I)

where R is alkyl of 1 to 8 carbon atoms ; phenyl optionally substituted by halogen atoms, alkyl groups or alkoxy groups ; or aralkyl, wherein a benzaldehyde dialkylacetal of the formula

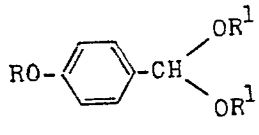

(II)

where $R^1$ is alkyl of 1 to 3 carbon atoms, is hydrogenated in water and in the presence of a transition metal of subgroup 8 as catalyst, at elevated temperature and under superatmospheric pressure.

2. A process as claimed in claim 1, wherein the hydrogenation is carried out at a temperature of from 40 to 140 °C and a pressure of from 20 to 400 bars.

3. A process as claimed in claim 1, wherein iron, cobalt or nickel is used as catalyst.

**Revendications**

1. Procédé pour la préparation d'alcools benzyliques de formule

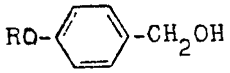

(I)

dans laquelle R représente un radical alcoyle à 1-8 atomes de C, un radical phényle, éventuellement substitué par des atomes d'halogène, des groupes alcoyle ou par des groupes alcoxy, ou un radical aralcoyle, caractérisé en ce qu'on hydrogène des acétals dialcoyliques de benzaldéhyde de formule

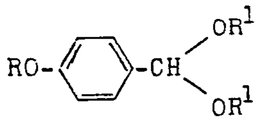

(II)

dans laquelle $R^1$ représente un radical alcoyle à 1-3 atomes de C, dans l'eau et en présence d'un métal de transition du groupe VIII servant de catalyseur, à haute température et sous pression élevée.

2. Procédé selon la revendication 1, caractérisé en ce qu'on réalise l'hydrogénation à températures de 40 à 140 °C et sous des pressions de 20 à 400 bars.

3. Procédé selon la revendication 1, caractérisé en ce qu'on utilise comme catalyseur du fer, du cobalt ou du nickel.